# DEMANDE DE BREVET EUROPEEN

(11) **EP 1 496 081 A1**
(43) Date de publication de la demande: **12.01.2005**
(21) Numéro de dépôt: 04291560.3
(22) Date de dépôt: 21.06.2004
(51) Int. Cl.: C08J 3/12, C08F 2/32

(54) **Nouvelle composition en poudre, procédé pour sa préparation et utilisation comme épaississant**

(30) Priorité: 26.06.2003 FR 0350261
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Mallo, Paul, 78290 Croissy sur Seine (FR)
(74) Mandataire: Conan, Philippe Claude

(57) **Abrégé**

Composition comprenant :
(a) - de 80 % à 99 % en poids d'au moins polyélectrolyte anionique, choisi parmi :
   - les homopolymères branchés ou réticulés d'un monomère possédant une fonction acide fort partiellement ou totalement salifiée,
   - les homopolymères branchés ou réticulés d'un monomère possédant une fonction acide faible partiellement ou totalement salifiée,
   - les copolymères branchés ou réticulés de monomères possédant une fonction acide fort partiellement ou totalement salifiée,
   - les copolymères branchés ou réticulés de monomères possédant une fonction acide faible partiellement ou totalement salifiée,
   - les copolymères branchés ou réticulés de monomères possédant une fonction acide fort partiellement ou totalement salifiée et soit d'au moins un monomère possédant une fonction acide faible partiellement ou totalement salifiée et/ou soit d'au moins un monomère neutre, et
   - les copolymères branchés ou réticulés de monomères possédant une fonction acide faible partiellement ou totalement salifiée et soit d'au moins un monomère neutre,
(b) - de 1 % à 20 % en poids d'au moins un agent émulsionnant de type eau-dans-huile (E/H), et
(c) - jusqu'à 19 % en poids d'eau.

Procédé pour sa préparation et applications en cosmétique.

## Description

L'invention a pour objet de nouveaux agents épaississants.

L'industrie cosmétique et l'industrie pharmaceutique utilisent très régulièrement des polymères épaississants synthétiques pour augmenter la viscosité des crèmes, des émulsions et de diverses solutions topiques. Les polymères épaississants synthétiques actuellement utilisés en ces domaines, se présentent sous deux formes physiques, la forme poudre et la forme liquide pour laquelle le polymère est dispersé dans une huile à l'aide d'agents tensioactifs et que l'on appelle couramment latex inverse.

Les polymères épaississants sous forme de poudre, les plus connus sont les polymères à base d'acide acrylique ou les copolymères à base d'acide acrylique et de ses esters. On citera par exemple les polymères commercialisés par la société Noveon sous le nom de marque, CARBOPOL™ et PEMULEN™. Ils sont décrits notamment dans les brevets américains US 5,373,044, US 2,798,053 et dans le brevet européen EP 0 301 532.

En cosmétique, on utilise aussi et toujours sous forme de poudre, des homopolymères ou copolymères à base d'acide 2-acrylamido-2-methylpropane sulfonique. Ces polymères épaississants sont commercialisés sous le nom de marque Aristoflex™ et décrits notamment dans les brevets européens EP 816 403, EP 1 116 733 et EP 1 069 142. Ces épaississants sous forme de poudre sont obtenus par polymérisation précipitante dans des solvants organiques, ce qui induit de nombreuses étapes successives d'épuration du produit final pour en élimer toute trace de solvant résiduel..

Les latex inverses auto inversibles ne présentent pas cet inconvénient et se dispersent très rapidement dans l'eau. Par contre, ils contiennent beaucoup d'huile et un ou plusieurs agents tensioactifs qui dans certains cas engendrent des réactions d'intolérance cutanée. Or, ce problème n'a encore été résolu de façon entièrement satisfaisante par le seul remplacement d'une huile par une autre. De plus, les latex inverses auto - inversibles ne permettent pas la préparation de gels clairs.

La demanderesse a donc cherché de nouveaux systèmes épaississants qui n'aient pas les inconvénients cités ci- dessus tout ayant des propriétés épaississantes suffisantes pour qu'ils soient une alternative acceptables aux compositions de l'état de la technique.

L'invention a pour objet une composition comprenant :
(a) de 80 % à 99 % en poids d'au moins polyélectrolyte anionique, choisi parmi :
   - les homopolymères branchés ou réticulés d'un monomère possédant une fonction acide fort partiellement ou totalement salifiée,
   - les homopolymères branchés ou réticulés d'un monomère possédant une fonction acide faible partiellement ou totalement salifiée,
   - les copolymères branchés ou réticulés de monomères possédant une fonction acide fort partiellement ou totalement salifiée,
   - les copolymères branchés ou réticulés de monomères possédant une fonction acide faible partiellement ou totalement salifiée,
   - les copolymères branchés ou réticulés de monomères possédant une fonction acide fort partiellement ou totalement salifiée et soit d'au moins un monomère possédant une fonction acide faible partiellement ou totalement salifiée et/ou soit d'au moins un monomère neutre, et
   - les copolymères branchés ou réticulés de monomères possédant une fonction acide faible partiellement ou totalement salifiée et soit d'au moins un monomère neutre,
(b) de 1 % à 20 % en poids d'au moins un agent émulsionnant de type eau-dans-huile (E/H), et
(c) jusqu'à 19 % en poids d'eau.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités, à bas gradient de vitesse, très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

La fonction acide fort du monomère en comportant est notamment la fonction acide sulfonique ou la fonction acide phosphonique, partiellement ou totalement salifiée. Ledit monomère peut être par exemple l'acide styrènesulfonique partiellement ou totalement salifié ou de préférence l'acide 2-méthyl 2-[(1-oxo 2-propènyl) amino] 1-propanesulfonique partiellement ou totalement salifié, notamment sous forme, soit d'un sel de métal alcalin tel que par exemple le sel de sodium ou le sel de potassium, soit d'un sel d'ammonium, soit le sel d'un amino alcool tel que par exemple le sel de monoéthanolamine ou soit le sel d'un aminoacide tel que par exemple le sel de lysine.

La fonction acide faible du monomère en comportant est notamment la fonction acide carboxylique, et de préférence, ledit monomère est choisi parmi l'acide acrylique, l'acide méthacrylique, l'acide itaconique, l'acide maléique ou l'acide 3-méthyl 3-[(1-oxo 2-propènyl) amino] butanoïque, lesdits acides étant partiellement ou totalement salifiés notamment sous forme, soit d'un sel de métal alcalin tel que par exemple le sel de sodium ou le sel de potassium, soit d'un sel d'ammonium, soit le sel d'un amino alcool tel que par exemple le sel de monoéthanolamine ou soit le sel d'un aminoacide tel que par exemple le sel de lysine.

Le monomère neutre est notamment choisi parmi l'acrylamide, le méthacrylamide, le diméthyl acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), le diacétone acrylamide ou un dérivé éthoxylé de poids moléculaire compris entre 400 et 1000, de chacun de ces esters.

Par "agent émulsionnant du type eau-dans-huile", on désigne des agents tensioactifs possédant une valeur de HLB suffisamment faible pour fournir des émulsions eau-dans-huile tels que les polymères tensioactifs du type copolymères séquencés polyéthylèneglycol poly(acide hydroxy stéarique), commercialisés par la demanderesse sous le nom de Simaline™ IE 200, tels que les esters de sorbitan, comme le monoléate de sorbitan commercialisé par la demanderesse sous le nom MONTANE™ 80, l'isostéarate de sorbitan commercialisé par la demanderesse sous le nom MONTANE™ 70, l'oléate de sorbitan éthoxylé avec 5 moles d'oxyde d'éthylène (5 OE) commercialisé par la demanderesse sous le nom MONTANE™ 81, l'alcool oléocétylique diéthoxylé (2 OE), commercialisé par la demanderesse sous le nom SIMULSOL™ OC 72 ou le sesquioléate de sorbitan commercialisé par la demanderesse sous le nom MONTANE™ 83.

Par « jusqu'à 19% en poids d'eau », on indique que la composition telle que définie précédemment, comprend une quantité non nulle d'eau.

La composition telle que définie précédemment, comprend souvent moins de 10 % en poids d'agent émulsionnant de type eau-dans-huile.

La composition telle que définie précédemment, comprend plus particulièrement au moins 0,5% en poids d'eau et fréquemment au moins 2 % en poids d'eau.

La composition telle que définie précédemment, comprend plus particulièrement moins de 10 % en poids d'eau.

La composition objet de la présente invention se présente sous forme de poudre.

Selon un premier mode particulier de la présente invention, la composition telle que définie précédemment comprend en outre :
(d) jusqu'à 19 % en poids en poids d'au moins un agent émulsionnant de type huile-dans-eau (H/E).

Par "agent émulsionnant du type huile-dans-eau", on désigne des agents tensioactifs possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène (20 OE), commercialisé par la demanderesse sous le nom MONTANOX™ 80, l'huile de ricin éthoxylée à 40 moles d'oxyde d'éthylène (40 OE), commercialisée par la demanderesse sous le nom SIMULSOL™ OL 50, le laurate de sorbitan éthoxylé à 20 moles d'oxyde d'éthylène (20 OE), commercialisé par la demanderesse sous le nom MONTANOX™ 20, le trioléate de sorbitan éthoxylé à 25 moles commercialisé par la demanderesse sous le nom MONTANOX™ 85, l'alcool laurique éthoxylé à 7 moles d'oxyde d'éthylène (7 OE), commercialisé par la demanderesse sous le nom SIMULSOL™ P 7, l'alcool oléocétylique décaéthoxylé (10 OE), commercialisé par la demanderesse sous le nom SIMULSOL™ OC 710 ou les hexaoléoates de sorbitan polyéthoxylés commercialisé sous les noms G-1086™ et G-1096™.

La composition telle que définie précédemment, comprend souvent moins de 10 % en poids d'agent émulsionnant de type huile-dans-eau.

Selon un deuxième mode particulier de la présente invention, la composition telle que définie précédemment comprend en outre :
(e) jusqu'à 19 % en poids d'huile.

Cette huile est généralement une huile minérale commerciale contenant des hydrocarbures saturés comme les paraffines, les isoparaffines ou les cycloparaffines, présentant à température ambiante, une densité entre 0,7 et 0,9 telles que par exemple, l'ISOPAR™ G, l'ISOPAR™ H, l'ISOPAR™ I, l'ISOPAR™ J, l'ISOPAR™ L, l'ISOPAR™ M, l'EXXOL™ D 100 S ou le MARCOL™ 52 commercialisés par EXXON CHEMICAL, l'isohexadécane ou l'isododécane, soit une huile de synthèse, soit par un mélange de plusieurs de ces huiles.

L'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER.

Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Le polyisobutène hydrogéné est commercialisé en France par la société Ets B. Rossow et Cie sous le nom PARLEAM - POLYSYNLANE™. Il est cité dans : Michel and Irene Ash ; Thesaurus of Chemical Products, Chemical Publishing Co, Inc. 1986, Volume I, page 211 (ISBN 0 7131 3603 0).

Le squalane est commercialisé en France par la société SOPHIM, sous le nom PHYTOSQUALAN™. Il est identifié dans Chemical Abstracts par le numéro RN = 111-01-3. C'est un mélange d'hydrocarbures contenant plus de 80% en poids de 2,6,10,15,19,23-hexaméthyl tétracosane.

La composition telle que définie précédemment, comprend souvent moins de 5 % en poids d'huile.

Selon un troisième mode particulier, l'invention a pour objet une composition telle que définie précédemment, caractérisée en ce que le polyélectrolyte anionique est réticulé et/ou branché avec un composé diéthylènique ou polyéthylènique dans la proportion molaire exprimée par rapport aux monomères mis en oeuvre, de 0,005% à 1%, et de préférence de 0,01 % à 0,5 % et, plus particulièrement de 0,01 % à 0,25 %, de préférence celle pour laquelle l'agent de réticulation et/ou l'agent de ramification est choisi parmi le diméthacrylate d'éthylèneglycol, l'acide diallyloxacétique ou un de ses sels comme le diallyloxyacétate de sodium, le diacrylate d'éthylèneglycol, le diallyl urée, le triallyl amine, le triméthylol propanetriacrylate ou le méthylène-bis(acrylamide) ou un mélange de ses composé

La composition telle que définie précédemment, peut également comprendre divers additifs tels que des agents complexants, des agents de transfert ou des agents limiteurs de chaîne.

L'invention a plus particulièrement pour objet la composition telle que définie précédemment, dans laquelle le polyélectrolyte anionique est choisi parmi les polymères suivants :
- Copolymère d'acide acrylique partiellement salifié sous forme de sel alcalin ou de sel d'ammonium et d'acrylamide, réticulé au méthylène-bis(acrylamide) ;
- Copolymère d'acide 2-méthyl 2-[(1-oxo 2-propènyl) amino] 1-propanesulfonique partiellement salifié sous forme de sel alcalin ou de sel d'ammonium et d'acrylamide, réticulé au méthylène-bis(acrylamide) ;
- Copolymère d'acide 2-méthyl 2-[(1-oxo 2-propènyl) amino] 1-propanesulfonique partiellement salifié sous forme de sel de sodium et d'acide acrylique partiellement salifié sous forme de sel alcalin ou de sel d'ammonium, réticulé au méthylène-bis(acrylamide) ;
- Copolymère d'acide 2-méthyl 2-[(1-oxo 2-propènyl) amino] 1-propanesulfonique partiellement salifié sous forme de sel alcalin ou de sel d'ammonium et d'acrylate de 2-hydroxy éthyle, réticulé au méthylène-bis(acrylamide) ;
- Homopolymère d'acide 2-méthyl 2-[(1-oxo 2-propènyl) amino] 1-propanesulfonique partiellement salifié sous forme de sel alcalin ou de sel d'ammonium réticulé au méthylène-bis(acrylamide) ;
- Homopolymère d'acide acrylique partiellement salifié sous forme de sel d'ammonium ou de sel de monoéthanol amine, réticulé au diallyloxyacétate de sodium ou
- Homopolymère d'acide acrylique partiellement salifié sous forme de sel d'ammonium ou de sel de monoéthanol amine, réticulé au triallyl amine.

L'invention a aussi pour objet un procédé de préparation de la composition telle que définie précédemment, caractérisé en ce que :
(a) l'on émulsionne une solution aqueuse contenant les monomères et les éventuels additifs, dans une phase huile en présence d'un ou plusieurs agents émulsionnants de type eau-dans-huile,
(b) l'on amorce la réaction de polymérisation par introduction dans l'émulsion formée en a), d'un initiateur de radicaux libres puis on la laisse se dérouler, et
(c) lorsque la réaction de polymérisation est terminée, l'émulsion de polymère formée est séchée par atomisation.

La technique d'atomisation ("spray - drying" en langue anglaise) consiste à créer un nuage de fines gouttelettes d'émulsions dans un courant d'air chaud pendant une durée contrôlée. On peut avantageusement utiliser un appareil de type Niro™.

Des équipements d'atomisation sont décrits en détail dans « Spray drying Handbook « by K. Master, 5th Ed, Longman Scientific 1991.

Selon une mise en oeuvre préférée du procédé tel que défini précédemment, la réaction de polymérisation est amorcée par un couple oxydo-réducteur, à une température inférieure ou égale à 20°C, puis conduite soit de manière quasi-adiabatique jusqu'à une température supérieure ou égale à 40°C, plus particulièrement supérieure ou égale à 50°C, soit en contrôlant l'évolution de la température.

L'invention a aussi pour objet une variante du procédé tel que défini précédemment, comprenant une étape (b') au cours de laquelle on ajoute dans le milieu réactionnel issu de l'étape (b), un ou plusieurs agents émulsionnants de type huile-dans-eau avant la mise en oeuvre de l'étape (c).

L'invention a encore pour objet un variante du procédé tel que défini précédemment, selon laquelle le milieu réactionnel issu de l'étape (b) ou de l'étape (b'), est concentré par distillation, avant la mise en oeuvre de l'étape (c).

L'invention a aussi pour objet un procédé de préparation de la composition telle que définie précédemment, caractérisé en ce que l'on atomise un latex inverse auto-inversible :
- de 15% à 40% en poids et de préférence de 20% à 25% en poids d'huile,
- de 20 % et 50 % d'eau,
- de 2,5% à 15% en poids, et de préférence de 4% à 9% en poids, d'agents émulsionnants, parmi lesquels de 20% à 50%, notamment de 25% à 40% du poids total des agents émulsionnants présents, sont du type eau-dans-huile (E/H) et parmi lesquels de 80% à 50%, notamment de 75% à 60%, du poids total des agents émulsionnants, sont du type huile-dans-eau (H/E) et
- de 20% à 70% en poids, et de préférence de 25% à 50% en poids, d'un polyélectrolyte anionique branché ou réticulé, choisi parmi :
   - les homopolymères branchés ou réticulés d'un monomère possédant une fonction acide fort partiellement ou totalement salifiée,
   - les homopolymères branchés ou réticulés d'un monomère possédant une fonction acide faible partiellement ou totalement salifiée,
   - les copolymères branchés ou réticulés de monomères possédant une fonction acide fort partiellement ou totalement salifiée,
   - les copolymères branchés ou réticulés de monomères possédant une fonction acide faible partiellement ou totalement salifiée,
   - les copolymères branchés ou réticulés de monomères possédant une fonction acide fort partiellement ou totalement salifiée et soit d'au moins un monomère possédant une fonction acide faible partiellement ou totalement salifiée et/ou soit d'au moins un monomère neutre, et
   - les copolymères branchés ou réticulés de monomères possédant une fonction acide faible partiellement ou totalement salifiée et soit d'au moins un monomère neutre,

Parmi les latex inverses auto-inversibles appropriés à la mise en oeuvre du procédé tel que défini ci-dessus, il y a par exemple les compositions commercialisées sous les marques :
SEPIGEL™ 305 (nom INCI : Polyacrylamide and C13-C14 Isoparaffin and Laureth-7),
SIMULGEL™ EG (nom INCI : Sodium acrylate and Acryloyldimethyl Taurate Copolymer and Isohexadecane and Polysorbate 80),
SIMULGEL™ NS (nom INCI : Hydroxyethyl Acrylate and Sodium Acryloyldimethyl Taurate Copolymer and Squalane and Polysorbate 60),
SIMULGEL™ A (nom INCI : Ammonium Polyacrylate and Isohexadecane and Polysorbate 89),
SIMULGEL™ 600 (nom INCI : Acrylamide and Sodium Acryloyldimethyl Taurate Copolymer and Polysorbate 80),
SIMULGEL™ 800 (nom INCI : Sodium Polyacryloyldimethyl Taurate and Isohexadecane and Sorbitan oleate),
SIMULGEL™ HT (Polyacrylamide and paraffin oil and Polysorbate 80), et
SIMULGEL EPG™ (nom INCI : Sodium Acrylate and Acryloyldimethyl Taurate copolymer and Polyisobutene and Caprylyl capryl Glucoside).

L'invention a aussi pour objet l'utilisation de la composition telle que définie précédemment comme épaississant et/ou comme émulsionnant, d'une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile-dans-eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte habituellement entre 0,1 % et 10 % en poids de l'agent épaississant défini ci-dessus. Le pH de la composition topique est de préférence supérieur ou égal à 5.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant conforme à l'invention mentionnée ci-dessus, pour épaissir et émulsionner une composition topique comprenant au moins une phase aqueuse.

La composition selon l'invention est un substitut intéressant à celles vendues sous le nom SEPIGEL™ 305 ou SEPIGEL™ 501, SIMULGEL™ EG, SIMULGEL™ NS SIMULGEL™ 600 ou SIMULGEL™ A par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL™ ou SIMULGEL™.

Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2 734 496, avec les agents tensioactifs décrits dans WO 93/08204.

Elle est particulièrement compatible avec le MONTANOV™ 68, le MONTA-NOV™ 82, le MONTANOV™ 202 ou le SEPIPERL™ N. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316.

Elle peut également être utilisée pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveux ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 19523596.

La composition selon l'invention est également compatible avec les principe actifs tels que par exemple, les agents auto-bronzants comme le dihydroxyacétone (DHA) ou les agents anti-acné; elle peut donc être introduite dans des compositions auto-bronzantes comme celles revendiquées dans EP 0 715 845, EP 0 604 249, EP 0 576 188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou WO 98/09611.

Les exemples suivants illustrent l'invention sans toutefois la limiter.

### Exemple A (exemple comparatif) : Préparation d'une composition A sans inverseur.

On charge dans un bêcher, sous agitation :
- 85,5 g d'eau permutée
- 95,0 d'une solution aqueuse d'hydroxyde de sodium à 48 % (en poids)
- 246,7 g de l'acide 2-methyl-2[(1-oxo-2 propènyl) amino] 1-propanesulfonique
- 253,8 g d'une solution aqueuse d'acrylamide à 50 % en poids
- 0,18 g de diéthylène triamine pentacétate de sodium,
- 0,61 g de triallyl amine et
- 0, 34 g persulfate d'ammonium. et

Le pH de la phase aqueuse précédemment décrit est ajusté à 5,2 et la quantité de phase aqueuse est complétée jusqu'à concurrence de 682 g par ajout d'eau permutée.

Parallèlement, on prépare une phase organique en introduisant dans un bêcher agité successivement :
- 220 g d'ISOPAR™ M
- 27,5 g de MONTANE™ 70 (iso stéarate de sorbitol commercialisé par Tepic)

La phase aqueuse est introduite progressivement dans la phase organique puis soumise à une agitation mécanique violente de type ultra-turrax™ commercialisé par IKA.

L'émulsion obtenue est alors transférée dans un réacteur de polymérisation. L'émulsion est soumise à un barbotage d'azote important de manière à éliminer l'oxygène et refroidit à environ 5-6°C.

Après un temps suffisant pour une bonne homogénéisation de la solution, on introduit alors une solution aqueuse de métabisulfite de sodium (0,2 g dans 100 ml d'eau) à raison de 0,5 ml/minute. L'introduction est réalisée pendant environ 60 minutes.

Pendant cette introduction, on laisse monter la température dans le réacteur de polymérisation jusqu'à la température finale de polymérisation.

On maintient alors le milieu réactionnel pendant environ 90 minutes à cette température.
L'ensemble est refroidi jusqu'à une température d'environ 35°C et l'on obtient la composition A.

### Exemple B (exemple comparatif) : Préparation d'une composition B avec inverseur.

On charge dans un bêcher, sous agitation
- 85,5 g d'eau permutée
- 95,0 d'une solution aqueuse d'hydroxyde de sodium à 48 % (en poids)
- 246,7 g de l'acide 2-methyl-2[(1-oxo-2 propènyl) amino] 1-propanesulfonique
- 253,8 g d'une solution aqueuse d'acrylamide à 50 % en poids
- 0,18 g de diéthylène triamine pentacétate de sodium
- 0,61 g de triallyl amine et
- 0, 34 g persulfate d'ammonium.

Le pH de la phase aqueuse précédemment décrit est ajusté à 5,2 et la quantité de phase aqueuse est complétée jusqu'à concurrence de 682 g par ajout d'eau permutée.

Parallèlement, on prépare une phase organique en introduisant dans un bêcher agité successivement :
- 220 g dISOPAR™ M
- 27,5 g de MONTANE™ 70 ( isostéarate de sorbitan commercialisé par SEPPIC)

La phase aqueuse est introduite progressivement dans la phase organique puis soumise à une agitation mécanique violente de type ultra-turrax™ commercialisé par IKA.

L'émulsion obtenue est alors transférée dans un réacteur de polymérisation. L'émulsion est soumise à un barbotage d'azote important de manière à éliminer l'oxygène et refroidit à environ 5-6°C.

Après un temps suffisant pour une bonne homogénéisation de la solution, on introduit alors une solution aqueuse de métabisulfite de sodium (0,2 g dans 100 ml d'eau) à raison de 0,5 ml/minute. L'introduction est réalisée pendant environ 60 minutes.

Pendant cette introduction, on laisse monter la température dans le réacteur de polymérisation jusqu'à la température finale de polymérisation.

On maintient alors le milieu réactionnel pendant environ 90 minutes à cette température.

L'ensemble est refroidi jusqu'à une température d'environ 35°C et l'on introduit lentement 35 g de Simulsol™ P7 ( Alcool laurique éthoxylé à 7 moles).

On obtient la composition B.

### Exemple 1 a) (exemple selon l'invention) : Préparation d'une composition 1 sans inverseur, selon l'invention.

On atomise la composition A obtenue à l'exemple A au moyen d'un appareil de type Niro™ et l'on obtient la composition 1 sous forme poudre.

### Exemple 1 b) (exemple selon l'invention) : Préparation d'une composition 2 avec inverseur, selon l'invention.

On atomise la composition B obtenue à l'exemple B au moyen d'un appareil de type Niro™ et l'on obtient la composition 2 sous forme poudre.

### Analyse et propriétés des compositions A, B, 1 et 2.

### Analyse

Les teneurs en chacun des constituants des compositions préparées sont consignées dans le tableau suivant :

| | Composition A | Composition 1 | Composition B | Composition 2 |
|---|---|---|---|---|
| Copolymère | 42,1% | 87% | 40,6% | 81,2% |
| Eau | 31,8% | 6,3% | 30,75% | 5,4% |
| ISOPAR M | 23,2% | 0,7% | 22,3% | 0,7% |
| MONTANE 7080 | 2,9% | 6 % | 2,8% | 5,6 % |
| SIMULSOL™ P7 | 0% | 0 % | 3,55 % | 7,1 % |
| | 100 % | 100 % | 100 % | 100 % |

### Propriétés

Les propriétés mises en évidence pour les compositions selon l'invention sont consignées dans le tableau suivant où elles sont aussi, mises en perspective avec celles des latex inverses auto-inversibles d'origine :

| | Composition A | Composition 1 | Composition B | Composition 2 |
|---|---|---|---|---|
| Viscosité à 2% dans l'eau¹ | 74 000 mPas Mobile : N° 6 Vitesse : 5 tours / minute | n.d. | 72 600 mPas Mobile: N°6 Vitesse : 5 tours / minute | n.d. |
| Viscosité à 2% dans de l'eau contenant 1% NaCl¹ | 2 760 mPas Mobile : N° 3 Vitesse : 5 tours / minute | n.d. | 1 440 mPas Mobile : N° 3 Vitesse : 5 tours / minute | n.d. |
| Viscosité à 1% dans l'eau | n.d. | 79 000 mPas Mobile : N° 6 Vitesse : 5 tours / minute | n.d. | 63400 mPas Mobile : N° 6 Vitesse : 5 tours / minute |
| Viscosité à 1% dans de l'eau | n.d. | 3 940 mPas Mobile : N° 3 | n.d. | 1 580 mPas N° 3 |
| contenant 1% NaCl | | Vitesse : 5 tours / minute | | Vitesse : 5 tours / minute |
| Aspect | gel blanc | Gel opalescent | | Gel clair |
| Temps de gélification1 | 1 minute | 1 minute | 1 minute | 1 minute |

| | | | | |
|---|---|---|---|---|
| 1 : les mesures de viscosité de la composition A (sans inverseur), sont effectuées dans une eau contenant du SIMULSOL™ P7. | | | | |

Ces résultats mettent en évidence que les compostions sous forme de poudre objets de la présente invention, ont des propriétés similaires à celles des latex inverses, tout étant plus aisées à mettre en oeuvre.

De plus un essai complémentaire a montré que la dispersion dans l'eau de la poudre du même polymère que celui présent dans les compositions 1 et 2, mais non associé à un quelconque tensioactif de type eau-dans-huile provoquait la formation d'un gel mais que l'expansion du polymère durait environ 24 heures.

Ceci démontre l'intérêt qu'il y à combiner un polymère, un tel tensioactif et de l'eau, au sein d'une même poudre atomisée pour exalter ses propriété épaississantes

Les exemples suivants mettent en oeuvre indifféremment les compositions 1 ou 2

### Exemple 2 : Crème de soin

| | |
|---|---|
| Cyclométhicone | 10% |
| Composition 1 | 0,8 % |
| MONTANOV™ 68 | 4,5 % |
| Conservateur | 0,65 % |
| Lysine | 0,025 % |
| EDTA (sel disodique) | 0,05 % |
| Gomme de xanthane | 0,2 % |
| Glycérine | 3% |
| Eau | qsp 100 % |

### Exemple 3 : Crème de soin

| | |
|---|---|
| Cyclométhicone | 10 % |
| Composition 1 | 0,8 % |
| MONTANOV™ 68 | 4,5 % |
| Perfluoropolymethylisopropylether | 0,5 % |
| Conservateur | 0,65 % |
| Lysine | 0,025 % |
| EDTA (sel disodique) | 0,05 % |
| PEMULEN™ TR | 0,2 % |
| Glycérine | 3 % |
| Eau | qsp 100% |

### Exemple 4 : Baume après-rasage

| FORMULE | | |
|---|---|---|
| A | Composition 1 | 1,5% |
| | Eau | q.s.p 100 % |
| | | |
| B | MICROPEARL™ M 100 | 5,0 % |
| | SEPICIDE™ CI | 0,50 % |
| | Parfum | 0,20 % |
| | Ethanol 95° | 10,0 % |

### MODE OPERATOIRE

Ajouter B dans A.

### Exemple 5 : Emulsion satinée pour le corps

| FORMULE | | |
|---|---|---|
| A | SIMULSOL™ 165 | 5,0 % |
| | LANOL™ 1688 | 8,50 % |
| | Beurre de Karité | 2 % |
| | Huile de paraffine | 6,5 % |
| | LANOL™ 14M | 3% |
| | LANOL™ S | 0,6 % |
| B | Eau | 66,2 % |
| | | |
| C | MICROPEARL™ M 100 | 5% |
| | | |
| D | Composition 1 | 3 % |
| | | |
| E | SEPICIDE™ CI | 0,3 % |
| | SEPICIDE™ HB | 0,5 % |
| | MONTEINE™ CA | 1 % |
| | Parfum | 0,20 % |
| | Acétate de vitamine E | 0,20 % |
| | Sodium pyrolidinone carboxylate | 1 % (agent hydratant) |

### MODE OPERATOIRE

Ajouter C dans B, émulsionner B dans A à 70°C, puis ajouter D à 60°C puis E à 30°C.

### Exemple 6 : Lait corporel

| FORMULE | | |
|---|---|---|
| A | SIMULSOL™ 165 | 5,0 % |
| | LANOL™ 1688 | 12,0 % |
| | LANOL™ 14M | 2,0 % |
| | Alcool cétylique | 0,3 % |
| | SCHERCEMOL™ OP | 3 % |
| B | Eau | q.s.p. 100% |
| C | Composition 1 | 0,35 % |
| D | SEPICIDE™ CI | 0,2 % |
| | SEPICIDE™ HB | 0,5 % |
| | Parfum | 0,20 % |

### MODE OPERATOIRE

Emulsionner B dans A vers 75°C ; ajouter C vers 60°C, puis D vers 30°C

### Exemple 7 : crème H/E

| FORMULE | | |
|---|---|---|
| A | SIMULSOL™ 65 | 5,0% |
| | LANOL™ 1688 | 20,0% |
| | LANOL™ P | 1,0% (additif à effet stabilisant) |
| | | |
| B | Eau | q.s.p. 100% |
| | | |
| C | Composition 1 | 2,50% |
| | | |
| D | SEPICIDE™ CI | 0,20% |
| | SEPICIDE™ HB | 0,30% |

### MODE OPERATOIRE

Introduire B dans A vers 75°C ; ajouter C vers 60°C, puis D vers 45°C

### Exemple 8 : gel solaire non gras

| FORMULE | | |
|---|---|---|
| A | Composition 1 | 3,00% |
| | Eau | 30% |
| | | |
| B | SEPICIDE™ C | 0,20% |
| | SEPICIDE™ HB | 0,30% |
| | Parfum | 0,10% |
| | | |
| C | Colorant | q.s. |
| | Eau | 30% |
| D | MICROPEARL™ M 100 | 3,00% |
| | Eau | q.s.p 100% |
| | | |
| E | Huile de silicone | 2,0% |
| | PARSOL™ MCX | 5,00% |

### MODE OPERATOIRE

Introduire B dans A; ajouter C puis D puis E.

### Exemple 9 : Lait solaire

| FORMULE | | |
|---|---|---|
| A | SEPIPERL™ N : | 3,0% |
| | Huile de sésame | 5,0% |
| | PARSOL™ MCX | 5,0% |
| | Carraghénane λ | 0,10% |
| | | |
| B | Eau | q.s.p.100% |
| | | |
| C | Composition 1 | 0,80% |
| | | |
| D | Parfum | q.s. |
| | Conservateur | q.s. |

### MODE OPERATOIRE

Emulsionner B dans A à 75°C puis ajouter C vers 60°C, puis D vers 30°C et ajuster le pH si nécessaire

### Exemple 10 : Gel de massage

| FORMULE | | |
|---|---|---|
| A | Composition 1 | 3,5% |
| | Eau | 20,0% |
| | | |
| B | Colorant | 2 gouttes/ 100g |
| | Eau | q.s. |
| | | |
| C | Alcool | 10% |
| | Menthol | 0,10% |
| | | |
| D | Huile de silicone | 5,0% |

### MODE OPERATOIRE

Ajouter B dans A; puis ajouter au mélange, C puis D

### Exemple 11 : gel soin de massage

| FORMULE | | |
|---|---|---|
| A | Composition 1 | 3,00% |
| | Eau | 30% |
| | | |
| B | SEPICIDE™ CI | 0,20% |
| | SEPICIDE™ HB | 0,30% |
| | Parfum | 0,05% |
| | | |
| C | colorant | q.s. |
| | Eau | q.s.p 100% |
| | | |
| D | MICROPEARL™ SQL | 5,0% |
| | LANOL™ 1688 | 2% |

### MODE OPERATOIRE

Préparer A; additionner B, puis C, puis D.

### Exemple 12 : Gel coup d'éclat

| FORMULE | | |
|---|---|---|
| A | Composition 1 | 4% |
| | Eau | 30% |
| | | |
| B | ELASTINE™ HPM | 5,0% |
| | | |
| C | MICROPEARL™ M 100 | 3% |
| | Eau | 5% |
| | | |
| D | SEPICIDE™ CI | 0,2% |
| | SEPICIDE™ HB | 0,3% |
| | Parfum | 0,06% |
| | Sodium pyrolidinone carboxylate 50% | 1% |
| | Eau | q.s.p. 100% |

### MODE OPERATOIRE

Préparer A; additionner B, puis C, puis D.

### Exemple 13 : Lait corporel

| FORMULE | | |
|---|---|---|
| A | SEPIPERL™ N | 3,0% |
| | Triheptonate de glycerol | 10,0% |
| | | |
| B | Eau | q.s.p.100% |
| | | |
| C | Composition 1 | 1,0% |
| D | Parfum | q.s. |
| | Conservateur | q.s. |

### MODE OPERATOIRE

Fondre A à environ 75°C . Emulsionner B dans A à 75°C puis ajouter C vers 60°C, puis D.

### Exemple 14 : Emulsion démaquillante à l'huile d'amande douce

| FORMULE | |
|---|---|
| MONTANOV™ 68 | 5% |
| Huile d'amandes douces | 5% |
| Eau | q.s.p.100% |
| Composition 1 | 0,3% |
| Glycérine | 5% |
| Conservateur | 0,2% |
| Parfum | 0,3% |

### Exemple 15 : Crème hydratante pour peaux grasses

| FORMULE | |
|---|---|
| MONTANOV™ 68 | 5% |
| Cétylstéaryloctanoate | 8% |
| Octyl palmitate | 2% |
| Eau | q.s.p.100% |
| Composition 1 | 0,6% |
| MICROPEARL™ M100 | 3,0% |
| Mucopolysaccharides | 5% |
| SEPICIDE™ HB | 0,8% |
| Parfum | 0,3% |

### Exemple 16 : Baume après-rasage apaisant sans alcool

| | |
|---|---|
| Mélange de lauryl aminoacides | 0,1% à 5% |
| Aspartate de magnésium et de potassium | 0,002% à 0,5% |
| LANOL™ 99 | 2% |
| Huile d'amandes douces | 0,5% |
| Eau | q.s.p.100% |
| Composition 1 | 3% |
| SEPICIDE™ HB | 0,3% |
| SEPICIDE™ CI | 0,2% |
| Parfum | 0,4% |

### Exemple 17 : Crème aux AHA pour peaux sensibles

| FORMULE | |
|---|---|
| Mélange de lauryl aminoacides | 0,1% à 5% |
| Aspartate de magnésium et de potassium | 0,002% à 0,5% |
| LANOL™ 99 | 2% |
| MONTANOV™ 68 | 5,0% |
| Eau | q.s.p.100% |
| Composition 1 | 1,50% |
| Acide gluconique | 1,50% |
| Triéthanolamine | 0,9% |
| SEPICIDE™ HB | 0,3% |
| SEPICIDE™ CI | 0,2% |
| Parfum | 0,4% |

### Exemple 18 : Soin apaisant après-soleil

| FORMULE | |
|---|---|
| Mélange de lauryl aminoacides | 0,1% à 5% |
| Aspartate de magnésium et de potassium | 0,002% à 0,5% |
| LANOL™ 99 | 10,0% |
| Eau | q.s.p.100% |
| Composition 1 | 2,50% |
| SEPICIDE™ HB | 0,3% |
| SEPICIDE™ CI | 0,2% |
| Parfum | 0,4% |
| Colorant | 0,03% |

### Exemple 19 : Lait démaquillant

| FORMULE | |
|---|---|
| SEPIPERL™N | 3% |
| PRIMOL™ 352: | 8,0% |
| Huile d'amandes douces: | 2% |
| Eau: | q.s.p.100% |
| Composition 1: | 0,8% |
| Conservateur: | 0,2% |

### Exemple 20 : Lait corporel

| FORMULE | |
|---|---|
| SEPIPERL™ N | 3,5% |
| LANOL™ 37T | 8,0% |
| SOLAGUM™ L | 0,05% |
| Eau | q.s.p.100% |
| Benzophénone | 2,0% |
| Diméthicone 350cPs | 0,05% |
| Composition 1 | 0,8% |
| Conservateur | 0,2% |
| Parfum | 0,4% |

### Exemple 21: émulsion fluide à pH alcalin

| | |
|---|---|
| MARCOL™ 82 | 5,0% |
| NaOH | 10,0% |
| Eau | q.s.p.100% |
| Composition 1 | 1,5% |

### Exemple 22 : Fond de teint fluide

| FORMULE | |
|---|---|
| SIMULSOL™ 165 | 5,0% |
| LANOL™ 84D | 8,0% |
| LANOL™ 99 | 5,0% |
| Eau | q.s.p.100% |
| Pigments et charges minérales | 10,0% |
| Composition 1 | 1,2% |
| Conservateur | 0,2% |
| Parfum | 0,4% |

### Exemple 23 : Lait solaire

| FORMULE | |
|---|---|
| SEPIPERL™N | 3,5% |
| LANOL™ 37T | 10,0% |
| PARSOL™ NOX | 5,0% |
| EUSOLEX™ 4360 | 2,0% |
| Eau | q.s.p. 100% |
| Composition 1 | 1,8% |
| Conservateur | 0,2% |
| Parfum | 0,4% |

### Exemple 24 : Gel contour des yeux

| FORMULE | |
|---|---|
| Composition 1 | 2,0% |
| Parfum | 0,06% |
| Sodium pyrrolidinonecarboxylate | 0,2% |
| DOW CORNING™ 245 FLuid | 2,0% |
| Eau | q.s.p. 100% |

### Exemple 25: composition de soin non rincée

| FORMULE | |
|---|---|
| Composition 1 | 1,5% |
| Parfum | q.s |
| Conservateur | q.s. |
| DOW CORNING™ X2 8360 | 5,0% |
| DOW CORNING™ Q2 1401 | 15% |
| Eau | q.s.p. 100% |

### Exemple 26: gel amincissant

| | |
|---|---|
| Composition 1 | 5 % |
| Ethanol | 30 % |
| Menthol | 0,1 % |
| Caféine | 2,5 % |
| Extrait de ruscus | 2 % |
| Extrait de lierre | 2 % |
| SEPICIDE™ HB | 1 % |
| Eau | q.s.p. 100 % |

### Exemple 27 : Baume après-rasage apaisant sans alcool

| FORMULE | | |
|---|---|---|
| A | LIPACIDE™ PVB | 1,0% |
| | LANOL™ 99 | 2,0% |
| | Huile d'amandes douces | 0,5% |
| | | |
| B | Composition 1 | 3,5% |
| | | |
| C | Eau | q.s.p. 100% |
| | | |
| D | Parfum | 0,4% |
| | SEPICIDE™ HB | 0,4% |
| | SEPICIDE™ CI | 0,2% |

### Exemple 28 : Gel rafraîchissant après-rasage

| FORMULE | | |
|---|---|---|
| A | LIPACIDE™ PVB | 0,5% |
| | LANOL™ 99 | 5,0% |
| | Composition 1 | 2,5% |
| | | |
| B | eau | q.s.p.100% |
| | | |
| C | MICROPEARL™ LM | 0,5% |
| | Parfum | 0,2% |
| | SEPICIDE™ HB | 0,3% |
| | SEPICIDE™ CI | 0,2% |

### Exemple 29: Soin pour les peaux grasses

| FORMULE | | |
|---|---|---|
| A | MICROPEARL™ M310 | 1,0% |
| | Composition 1 | 5,0% |
| | Isononanoate d'octyle | 4.0% |
| | | |
| B | Eau | q.s.p.100% |
| | | |
| C | SEPICONTROL™ A5 | 4,0% |
| | Parfum | 0,1% |
| | SEPICIDE™ HB | 0,3% |
| | SEPICIDE™ CI | 0,2% |
| | | |
| D | CAPIGEL™ 98 | 0,5% |
| | Eau | 10% |

### Exemple 30 : Crème aux AHA

| FORMULE | | |
|---|---|---|
| A | MONTANOV™ 68 | 5,0% |
| | LIPACIDE™ PVB | 1,05% |
| | LANOL™ 99 | 10,0% |
| | | |
| B | Eau | q.s.p. 100% |
| | Acide gluconique | 1,5% |
| | TEA (triéthanolamine) | 0,9% |
| | | |
| C | Composition 1 | 1,5% |
| | | |
| D | Parfum | 0,4% |
| | SEPICIDE™ HB | 0,2% |
| | SEPICIDE™ CI | 0,4% |

### Exemple 31 : Autobronzant non gras pour visage et corps

| FORMULE | | |
|---|---|---|
| A | LANOL™ 2681 | 3,0% |
| | Composition 1 | 2,5% |
| | | |
| B | Eau | q.s.p.100% |
| | Dihydroxyacétone | 3,0% |
| | | |
| C | Parfum | 0,2% |
| | SEPICIDE™ HB | 0,8% |
| | NaOH (hydroxyde de sodium) | qs pH = 5 % |

### Exemple 32 : Lait solaire au monoï de Tahiti

| FORMULE | | |
|---|---|---|
| A | Monoï de Tahiti | 10% |
| | LIPACIDE™ PVB | 0,5% |
| | Composition 1 | 2,2% |
| B | Eau | q.s.p. 100% |
| | | |
| C | Parfum | 0,1% |
| | SEPICIDE™ HB | 0,3% |
| | SEPICIDE™ CI | 0,1% |
| | Méthoxycinnamate d'octyle | 4,0% |

### Exemple 33 : Soin solaire pour le visage

| FORMULE | | |
|---|---|---|
| A | Cyclométhicone et diméthiconol | 4,0% |
| | Composition 1 | 3,5% |
| | | |
| B | Eau | q.s.p. 100% |
| | | |
| C | Parfum | 0,1 % |
| | SEPICIDE™ HB | 0,3% |
| | SEPICIDE™ CI | 0,21% |
| | Méthoxycinnamate d'octyle | 5,0% |
| | Micatitane | 2,0% |
| | Acide lactique | q.s.p. pH = 6,5 |

### Exemple 34 : Emulsion bronzante sans soleil

| FORMULE | | |
|---|---|---|
| A | LANOL™ 99 | 15% |
| | MONTANOV™ 68 | 5,0% |
| | Paraméthoxycinnamate d'octyle | 3,0% |
| | | |
| B | Eau | q.s.p. 100% |
| | Dihydroxyacétone | 5,0% |
| | Phosphate monosodique | 0,2% |
| C | Composition 1 | 0,5% |
| | | |
| D | Parfum | 0,3% |
| | SEPICIDE™ HB | 0,8% |
| | NaOH | q.s. pH=5. |

### Exemple 35 : Gel brillance

| | |
|---|---|
| Composition 1 | 1,5 % |
| Silicone volatil | 25 % |
| Monopropylèneglycol | 25 % |
| Eau déminéralisée | 10 % |
| Glycérine | qsp 100 % |

### Exemple 36 : Gel amincissant

| | |
|---|---|
| Composition 1 | 1,5 % |
| Isononylisononanoate | 2 % |
| Caféine | 5 % |
| Ethanol | 40 % |
| MICROPEARL™ LM | 2 % |
| Eau déminéralisée | qsp 100 % |
| Conservateur parfum | qs |

### Exemple 37 : Lait démaquillant

| | |
|---|---|
| SIMULSOL™ 165 | 4% |
| MONTANOV™ 202 | 1 % |
| Caprylate-caprate triglycéride | 15 % |
| PECOSIL™ DCT | 1 % |
| Eau déminéralisée | qs |
| CAPIGEL™ 98 | 0,5 % |
| Composition 1 | 1% |
| PROTEOL™ OAT | 2 % |
| NaOH | qsp pH 7 |

### Exemple 38 : Crème solaire

| | |
|---|---|
| SIMULSOL™ 165 | 3 % |
| MONTANOV™ 202 | 2 % |
| Benzoate C12-C15 | 8% |
| PECOSIL™ PS 100 | 2 % |
| Diméthicone | 2 % |
| Cyclométhicone | 5 % |
| Octyl-méthoxy-cinnamate | 6 % |
| Benzophénone-3 | 4 % |
| Oxyde de Titane | 8 % |
| Gomme xanthane | 0,2 % |
| Butylène-glycol | 5 % |
| Eau déminéralisée | qsp 100 % |
| Composition 1 | 1,5 % |
| Conservateur, parfum | qs |

### Exemple 39 : Gel de soin peaux mixtes

| | |
|---|---|
| Composition 1 | 4% |
| Squalane végétal | 5 % |
| Dimethicone | 1,5 % |
| SEPICONTROL™ A5 | 4 % |
| Gomme xanthane | 0,3 % |
| Eau | qsp 100 % |
| Conservateur, Parfum | qs |

### Exemple 40 : Voile parfumé pour le corps

| | |
|---|---|
| Composition 1 | 1,5% |
| Cyclométhicone | 5 % |
| Parfum | 2 % |
| MICROPEARL™ M100 | 5 % |
| Glycérine | 5% |
| Eau déminéralisée | qsp 100 % |

### Exemple 41 : Crème vitaminée

| | |
|---|---|
| SIMULSOL™ 165 | 5% |
| MONTANOV™ 202 | 1 % |
| Caprylic/capric triglycerides | 20 % |
| Palmitate de vitamine A | 0,2 % |
| Acetate de vitamine E | 1 % |
| MICROPEARL™ M305 | 1,5 % |
| Composition 1 | 0,7 % |
| Eau | qsp 100% |
| Conservateur, parfum | qs |

Le MONTANOV™ 68 (cétéaryl glucoside), est une composition auto-émulsionnable telle que décrite dans WO 92/06778, commercialisée par la société SEPPIC.
Le MICROPEARL™ M 100 est une poudre ultra fine au toucher très doux et à action matifiante commercialisée par la société MATSUMO
Le SEPICIDE™ CI, imidazolidine urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL™ S sont des facteurs de consistance commercialisés par la société SEPPIC.
Le SEPICIDE™ HB , qui est un mélange de phénoxyéthanol, de méthyl paraben, d'éthylparaben, de propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le PARSOL™ MCX est de l'octyl paraméthoxycinnamate; commercialisé par la société GIVAUDAN.
Le SEPIPERL™ N est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage; il est commercialisé par la société MATSUMO.
Le LANOL™ 99 est de l'isononyl isononanoate commercialisé par la société SEPPIC.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société EXXON.
Le LANOL™ 84D est du malate de dioctyle commercialisé par la société SEPPIC.
Le PARSOL™ NOX est un filtre solaire commercialisé par la société GIVAUDAN.
L'EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB, est un hydrolysat de protéines de blé acylé commercialisé par la société SEPPIC.
Le MICROPEARL™ LM est un mélange de squalane, de polyméthylméthacrylate et de menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère acrylique commercialisé par la société SEPPIC.
Le LANOL™ 2681 est un mélange caprylate, caprate de coprah, commercialisé par la société SEPPIC.
Le MONTANOV™ 202, est une composition APG/alcools gras telle que décrite dans WO9 98/47610, commercialisée par la société SEPPIC.

## Revendications

1. Composition comprenant :
(a) - de 80 % à 99 % en poids d'au moins polyélectrolyte anionique, choisi parmi :
- les homopolymères branchés ou réticulés d'un monomère possédant une fonction acide fort partiellement ou totalement salifiée,
- les homopolymères branchés ou réticulés d'un monomère possédant une fonction acide faible partiellement ou totalement salifiée,
- les copolymères branchés ou réticulés de monomères possédant une fonction acide fort partiellement ou totalement salifiée,
- les copolymères branchés ou réticulés de monomères possédant une fonction acide faible partiellement ou totalement salifiée,
- les copolymères branchés ou réticulés de monomères possédant une fonction acide fort partiellement ou totalement salifiée et soit d'au moins un monomère possédant une fonction acide faible partiellement ou totalement salifiée et/ou soit d'au moins un monomère neutre, et
- les copolymères branchés ou réticulés de monomères possédant une fonction acide faible partiellement ou totalement salifiée et d'au moins un monomère neutre ;
(b) - de 1 % à 20 % en poids d'au moins un agent émulsionnant de type eau-dans-huile (E/H), et
(c) - jusqu'à 19 % en poids d'eau.

2. Composition telle que définie à la revendication 1, **caractérisée en ce qu'**elle comprend moins de 10 % en poids d'agent émulsionnant de type eau-dans-huile.

3. Composition telle que définie à l'une des revendications ou 2, **caractérisée en ce qu'**elle comprend au moins 0,5% en poids d'eau et fréquemment au moins 2 % en poids d'eau.

4. Composition telle que définie à la revendication 3, **caractérisée en ce qu'**elle comprend au moins 2 % en poids d'eau.

5. Composition telle que définie à l'une des revendications 1 à 4, **caractérisée en ce qu'**elle comprend moins de 10 % en poids d'eau.

6. Composition telle que définie à l'une des revendications 1 à 5, **caractérisée en ce qu'**elle comprend en outre :
(d) - jusqu'à 19 % en poids, d'au moins un agent émulsionnant de type huile-dans-eau (H/E).

7. Composition telle que définie à la revendication 6, **caractérisée en ce qu'**elle comprend moins de 10 % en poids d'agent émulsionnant de type huile-dans-eau.

8. Composition telle que définie à l'une des revendications 1 à 7, **caractérisée en ce qu'**elle comprend en outre :
(e) - jusqu'à 19 % en poids d'huile.

9. Composition telle que définie à la revendication 8, **caractérisée en ce que** l'huile est choisie parmi les huiles blanches minérales, le squalane, le polyisobutène hydrogéné, le palmitate d'octyle, l'isostéarate d'isostéaryle, l'isododécane ou l'isohexadécane.

10. Composition telle que définie à l'une des revendications 8 ou 9, **caractérisée en ce qu'**elle comprend moins de 5 % en poids d'huile.

11. Composition telle que définie à l'une des revendications 1 à 10, **caractérisée en ce que** le polyélectrolyte anionique est réticulé et/ou branché avec un composé diéthylènique ou polyéthylènique dans la proportion molaire exprimée par rapport aux monomères mis en oeuvre, de 0,005% à 1%, et de préférence de 0,01 % à 0,5 % et, plus particulièrement de 0,01 % à 0,25 %, de préférence celle pour laquelle l'agent de réticulation et/ou l'agent de ramification est choisi parmi le diméthacrylate d'éthylèneglycol, l'acide diallyloxacétique ou un de ses sels comme le 1 diallyloxyacétate de sodium, le diacrylate d'éthylèneglycol, le diallyl urée, le triallyl amine, le triméthylol propanetriacrylate ou le méthylènebis-(acrylamide) ou un mélange de ses composés.

12. Composition telle que définie à l'une des revendications 1 à 11, dans laquelle le polyélectrolyte anionique est choisi parmi les polymères suivants :
- Copolymère d'acide acrylique partiellement salifié sous forme de sel de sodium et d'acrylamide, réticulé au méthylène bis(acrylamide) ;
- Copolymère d'acide 2-méthyl 2-[(1-oxo 2-propènyl) amino] 1-propanesulfonique partiellement salifié sous forme de sel de sodium et d'acrylamide, réticulé au méthylène bis(acrylamide) ;
- Copolymère d'acide 2-méthyl 2-[(1-oxo 2-propènyl) amino] 1-propanesulfonique partiellement salifié sous forme de sel de sodium et d'acide acrylique partiellement salifié sous forme de sel de sodium, réticulé au méthylène bis(acrylamide)
- Copolymère d'acide 2-méthyl 2-[(1-oxo 2-propènyl) amino] 1-propanesulfonique partiellement salifié sous forme de sel de sodium et d'acrylate de 2-hydroxy éthyle, réticulé au méthylène bis(acrylamide) ;
- Homopolymère d'acide 2-méthyl 2-[(1-oxo 2-propènyl) amino] 1-propanesulfonique partiellement salifié sous forme de sel de sodium réticulé au méthylène bis(acrylamide) ;
- Homopolymère d'acide acrylique partiellement salifié sous forme de sel d'ammonium ou de sel de monoéthanol amine, réticulé au diallyloxyacétate de sodium ou
- Homopolymère d'acide acrylique partiellement salifié sous forme de sel d'ammonium ou de sel de monoéthanol amine, réticulé au triallyl amine.

13. Procédé de préparation de la composition telle que définie à la revendication 1 à 12, **caractérisé en ce que** :
a) l'on émulsionne une solution aqueuse contenant les monomères et les éventuels additifs, dans une phase huile en présence d'un ou plusieurs agents émulsionnants de type eau-dans-huile,
b) l'on amorce la réaction de polymérisation par introduction dans l'émulsion formée en a), d'un initiateur de radicaux libres puis on la laisse se dérouler,
c) lorsque la réaction de polymérisation est terminée, l'émulsion est séchée par atomisation.

14. Variante du procédé tel que défini à la revendication 13, comprenant une étape (b') au cours de laquelle on ajoute dans le milieu réactionnel issu de l'étape b), un ou plusieurs agents émulsionnants de type huile-dans-eau avant la mise en oeuvre de l'étape (c).

15. Variante du procédé tel que défini à l'une des revendications 13 ou 14, selon laquelle le milieu réactionnel issu de l'étape (b) ou de l'étape (b'), est concentré par distillation, avant la mise en oeuvre de l'étape (c).

16. Procédé de préparation de la composition telle que définie à l'une des revendications 1 à 12, **caractérisé en ce que** l'on atomise un latex inverse auto-inversible :
- de 15% à 40% en poids et de préférence de 20% à 25% en poids d'huile,
- de 20 % et 50 % d'eau,
- de 2,5% à 15% en poids, et de préférence de 4% à 9% en poids, d'agents émulsionnants, parmi lesquels de 20% à 50%, notamment de 25% à 40% du poids total des agents émulsionnants présents, sont du type eau-dans-huile (E/H) et parmi lesquels de 80% à 50%, notamment de 75% à 60%, du poids total des agents émulsionnants, sont du type huile-dans-eau (H/E) et
- de 20% à 70% en poids, et de préférence de 25% à 50% en poids, d'un polyélectrolyte anionique branché ou réticulé, choisi parmi :
- les homopolymères branchés ou réticulés d'un monomère possédant une fonction acide fort partiellement ou totalement salifiée,
- les homopolymères branchés ou réticulés d'un monomère possédant une fonction acide faible partiellement ou totalement salifiée,
- les copolymères branchés ou réticulés de monomères possédant une fonction acide fort partiellement ou totalement salifiée,
- les copolymères branchés ou réticulés de monomères possédant une fonction acide faible partiellement ou totalement salifiée,
- les copolymères branchés ou réticulés de monomères possédant une fonction acide fort partiellement ou totalement salifiée et soit d'au moins un monomère possédant une fonction acide faible partiellement ou totalement salifiée et/ou soit d'au moins un monomère neutre, et
- les copolymères branchés ou réticulés de monomères possédant une fonction acide faible partiellement ou totalement salifiée et soit d'au moins un monomère neutre.

17. Utilisation de la composition telle que définie à l'une des revendications 1 à 12, comme épaississant et/ou émulsionnant d'une composition topique cosmétique, dermo-pharmaceutique ou pharmaceutique.
